# EUROPEAN PATENT APPLICATION

(11) **EP 2 229 876 A1**
(43) Date of publication of application: **22.09.2010**
(21) Application number: 10002680.6
(22) Date of filing: 15.03.2010
(51) Int. Cl.: A61B 5/00, A61B 5/155, G06F 19/00

(54) **Wirelessly configurable medical device for a broadcast network system**

(30) Priority: 16.03.2009 US 404498
(71) Applicant: Roche Diagnostics GmbH, 68305 Mannheim (DE); F.HOFFMANN-LA ROCHE AG, 4070 Basel (CH)
(72) Inventor: Buck, Harvey, Indianapolis, IN 46256 (US); Beaty, Terry, Indianapolis, IN 46278 (US)

(57) **Abstract**

A system for configuring one or more medical devices used for patient self-monitoring of medical parameters includes a broadcast provider and one or more medical devices in a network area of the broadcast provider. The one or more medical devices each include a receiver operable to receive data signals transmitted over the network area from the broadcast provider. The signals transmit medical device data to the one or more medical devices to facilitate the use of the medical device by the user while reducing the potential for errors that may be made by the user when using the medical device.

## Description

As the number of patients suffering from diabetes and other medical conditions increases, self-monitoring of physical parameters, such as where the patient monitors his or her blood glucose levels, has become a common practice. In the case of diabetics, the purpose of monitoring the blood glucose level is to determine the concentration level and then to take corrective action, based upon whether the level is too high or too low, to bring the level back within a normal range. The failure to take corrective action can have serious medical implications. Glucose monitoring is a fact of everyday life for diabetic individuals. Failure to test blood glucose levels properly and on a regular basis can result in serious diabetes-related complications, including cardiovascular disease, kidney disease, nerve damage and blindness. Monitoring of other physical parameters by patients with other conditions can be similarly important.

When medical devices are used by the patient or other user for self-monitoring, it can be difficult to quickly and accurately provide update information to the patient once the medical device is under the control of the end user. For example, product updates, recalls, upgrades in programming or memory could be forwarded to the patient by electronic mail, postal service, or the world-wide-web, but receiving such information typically requires some action by the user to receive the data and place it into the medical device. Even if the patient takes such action, the update to the medical device may not be done promptly or correctly by the user, increasing the potential for improper and inaccurate self-monitoring of medical parameters with the medical device. In addition, communications by the manufacturer with the user after the medical device is under the control of the user can be costly if, for example, conducted by mail or phone. Communications by electronic mail or through the world-wide-web may not reach the user if the manufacturer database of user contact information is inaccurate, incomplete, out-of-date, or if the user does not access the world-wide-web or act on any instructions contained in electronic mail.

In the example of blood glucose meters, a number of glucose meters are currently available that permit an individual to test the glucose level in a small sample of blood. Many of the meter designs currently available make use of a disposable test element which in combination with the meter measures the amount of glucose in the blood sample electrochemically. In order to take accurate measurements with blood glucose test elements, it is necessary to calibrate the meter to the tests strips in the vial or package from which the test element will be taken. However, current techniques for providing calibration information relating to a particular lot of test elements suffer several drawbacks. The user may be burdened to enter a code into the meter that provides calibration information for each test element vial that will be used. If the user does not enter the code or enters an incorrect code, then blood glucose readings may be compromised. Other techniques burden the manufacturer of the test elements since the calibration information is determined after manufacture of the test elements, and additional handling and packaging is required to place the calibration code on the strips or to provide a separate key with each vial having the calibration information thereon. Other techniques pre-determine the calibration information from the material used to make the test elements. However, this requires precisely controlling manufacture of the test elements and has the risk that the material is not adequately sampled or that the predetermined code is changed during the manufacturing and packaging process.

Another technique is to provide a wireless network comprising two-way communication between a meter and a centralized data manager, such as is employed by the ACCU-CHEK® Inform and Inform II systems in the hospital setting. Using this technique, the meter identifies itself to the wireless network and is then configured or downloaded with appropriate information in accordance with the centralized data manager. The two-way communication is initiated by the meter, which contains various registry keys that are indicative of its present configured state. If the data manager determines the meter needs updating, it will force a download and update the relevant keys in the meter. For simplicity, all codes in use are typically pushed to, i.e. stored, in the meter. However, it is still required that a user of the meter specifically indicates to the meter a lot or code number that it then correlates to the information received from the centralized data manager.

Given the ramifications of accurate recording and reporting of physical parameters for monitoring and treatment of medical conditions, improvements in the apparatus and/or procedures to maintain the medical devices with up-to-date programming, data and patient information are desired.

A medical device for patient self-monitoring of medical parameters includes a receiver operable to seamlessly receive data signals transmitted over a broadcast network system from a broadcast provider with no user intervention or interaction with the medical device. The signals transmit medical device data to the medical device that updates the memory or configuration of the medical device without requiring the user to interact with the medical device.

In one aspect, a system for remotely configuring medical devices comprises a plurality of portable medical devices distributed over a network area. Each of the medical devices includes a receiver to receive data signals when the data signals are broadcast in the network area, and each of the medical devices further includes a memory and a processor operably linked with the receiver. The system also includes a broadcast network system for broadcasting medical device data transmitted by the data signals over the network area for receipt by the receivers of each of the plurality of medical devices in the network area. The plurality of medical devices each include means operable upon receiving the data signals for at least one of updating operating parameters of the processor in accordance with the medical device data and storing the medical device data in the memory.

In one refinement of the aspect, the broadcast network system includes a broadcast provider linked to the broadcast network system, and a medical device manufacturer for supplying the medical device data to the broadcast provider. The broadcast provider formats the medical device data for wireless transmission in the network area.

In another refinement of the aspect, the system includes a communications link between the medical device manufacturer and the broadcast provider, and the medical device data is formatted by the broadcast provider by at least one of encoding, compression, and packetization of the medical device data for broadcast with the data signals over the network area.

In another refinement of the aspect, the medical device data include calibration data for at least one lot of analyte test elements.

In another refinement of the aspect, the calibration data includes a test element lot number and calibration parameters associated with the test element lot number.

In another refinement of the aspect, at least a portion of the plurality of medical devices receive the data signals and store the calibration data associated with the data signals in the memory thereof. The processors of the portion of the medical devices are operable to automatically associate the stored test element lot number to a lot number of an analyte test element detected by the medical devices and calibrate the medical devices with the calibration parameters of the associated test element lot number.

In another refinement of the aspect, the memory of the portion of the medical devices includes calibration data received from the broadcast network system for a plurality of lots of analyte test elements.

In another refinement of the aspect, the medical device data includes at least one of treatment information, medical device information, warning information, promotional information, and motivational information.

In another refinement of the aspect, the processor is operable to display the medical device data on the medical device when the medical device is accessed by the user and the medical device data is stored in the memory of the medical device.

In another refinement of the aspect, the broadcast network system includes either at least one satellite transmitter or at least one local transmitter.

In another refinement of the aspect, the receiver is operable to automatically receive the data signals when the data signals are broadcast over the network area and the medical device is in the network area.

In another refinement of the aspect, the processor is operable to automatically perform an operation with the medical device data transmitted with the data signals without user interaction with the medical device.

In a further aspect, a portable blood glucose meter comprises a housing including a display and a user entry means for receiving user input; a memory and a processor in the housing operably connected to the display and the user entry means; a port including circuitry for determining a value representative of a property of a fluid sample carried by an analyte test element inserted in the port and providing the value to the processor; and a receiver operable to seamlessly receive data signals from a broadcast network system. The receiver is linked to the processor and the memory and the processor is operable to seamlessly process medical device data transmitted with the data signals from the receiver in order to at least one of update operating parameters of the processor with the medical device data and store the medical device data in the memory.

In one refinement of the aspect, the receiver includes a decoder and the receiver and decoder are operable to receive and decode the data signals.

In another refinement of the aspect, the processor is operable to update operating instructions of the processor with the medical device data transmitted with the data signals.

In another refinement of the aspect, the processor is operable to update software stored in the memory with the medical device data transmitted with the data signals.

In another refinement of the aspect, the medical device data transmitted with the data signals includes calibration data for at least one lot of analyte test elements and the processor is operable to store the calibration data in the memory.

In another refinement of the aspect, the processor is operable to associate the calibration data stored in the memory to an analyte test element inserted in the port.

In another aspect, a method for updating a medical device comprises providing a broadcast network system with a network area; broadcasting data signals over the broadcast network system to a plurality of medical devices in the network area, wherein each of the plurality of medical devices receives the data signals; interrogating the data signals with each of the medical devices; and performing an operation with each of the medical devices based on the interrogation of the data signals.

In one refinement of the aspect, performing the operation with each of the medical devices includes at least a portion of the medical devices disregarding the data signals.

In another refinement of the aspect, performing the operation with each of the medical devices includes at least a portion of the medical devices updating an operating system thereof with medical device data transmitted with the data signals.

In another refinement of the aspect, performing the operation with each of the medical devices includes at least a portion of the medical devices storing medical device data transmitted with the data signals in a memory of the portion of the medical devices.

In another refinement of the aspect, performing the operation with each of the medical devices includes at least one of: disregarding the data signals, updating an operating system of the medical devices with medical device data transmitted with data signals, and storing medical device data transmitted with the data signals in a memory of the medical devices.

In another refinement of the aspect, each of the plurality of medical devices seamlessly receives the data signals when the medical device is located in the network area.

In another refinement of the aspect, each of the plurality of medical devices seamlessly receives the data signals when the medical device is located in the network area.

In another refinement of the aspect, the data signals are formatted for wireless transmission over the network area.

In another refinement the aspect, the method further includes providing medical device data to a broadcast provider, wherein the medical device data is provided by a manufacturer of the plurality of medical devices; and formatting the medical device data for transmission over the broadcast network system with the data signals.

In another refinement of the aspect, the data signals include calibration data for a manufactured lot of analyte test elements.

In another refinement of the aspect, the calibration data includes one or more of an analyte test element lot number and calibration parameters associated with the analyte test element lot number.

In another refinement of the aspect, the method further comprises storing the calibration data in a memory of at least a portion of the plurality of medical devices; associating the stored analyte test element lot number to a lot number of an analyte test element detected by a corresponding one of the portion of medical devices; calibrating the corresponding medical device with the calibration parameters of the associated stored analyte test element lot number; and testing an analyte with the detected analyte test element using the calibrated medical device.

In another refinement of the aspect, the method further comprises storing the calibration data in a memory of at least a portion of the plurality of medical devices; associating the stored analyte test element lot number to a lot number of an analyte test element detected by a corresponding one of the portion of medical devices; calibrating the corresponding medical device with the calibration parameters of the associated stored analyte test element lot number; and testing an analyte with the detected analyte test element using the calibrated medical device.

In another refinement of the aspect, the memory of the calibrated medical device includes calibration data for a plurality of lots of analyte test elements and the associated stored analyte test element lot number is selected from the calibration data of the plurality of lots of analyte test elements stored in the memory.

In another refinement of the aspect, the data signals include at least one of expiration data for a lot of analyte test elements and recall data for a lot of analyte test elements.

In another refinement of the aspect, medical device data is transmitted by the data signals and includes at least one of treatment information, medical device information, warning information, promotional information, and motivational information; performing the operation with each of the medical devices includes at least a portion of the medical devices storing the medical device data transmitted with the data signals in a memory of the portion of the medical devices, and further comprising displaying the medical device data on a display of a corresponding one of the medical devices when subsequently accessed by a user of the medical device.

In another refinement of the aspect, the data signals include at least one medical device identification number to which the data signals are directed

These and other aspects are also discussed below.
**Fig. 1** is a flowchart showing a sequence for broadcasting data provided by a manufacturer of medical devices to a broadcast provider for broadcast over a network area to a plurality of medical devices.
**Fig. 2** is a diagram showing a broadcast provider and a plurality of medical devices in a network area of the broadcast provider to receive data signals from the broadcast provider.
**Fig. 3** is a flowchart showing a method for broadcasting data signals over a network area to a plurality of medical devices in the network area.
**Fig. 4** is a flowchart showing a method for configuring medical devices with data transmitted by data signals from the broadcast provider.
**Fig. 5** is a plan view of one embodiment medical device for receiving data signals from a broadcast provider.
**Fig. 6** is a schematic of the medical device of Fig. 5.

For purposes of promoting an understanding of the principles of the invention, reference will now be made to the embodiments illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

The present invention includes at least one medical device with a receiver operable to receive data signals from a broadcast network system that transmits medical device data to the at least one medical device. The medical device data can be used by the medical device to wirelessly configure or update the medical device for operation by the user. In one aspect, the at least one medical device is mobile and is remote from a central and stationary broadcast location from which medical device data is transmitted over the network area. In another aspect, the medical device data is broadcast using a radio frequency broadcast system to provide a wide coverage area and a signal that is transmitted to the medical device regardless of the location of the medical device so long as it is within the network area. In still another aspect, the at least one medical device is mobile and is remote from a satellite broadcast location from which medical device data is transmitted over the network area. Configuring the medical device with the medical device data occurs automatically and does not require user interaction with the medical device, ensuring the medical device includes updated operating parameters and updated medical device data in its memory when used by the user.

Specific examples are provided herein of an embodiment that includes a blood glucose meter. It should be understood, however, that the present invention has application with other medical devices used for self-monitoring of other medical parameters. In one specific example, the present invention provides a broadcast system to broadcast calibration data for an analyte testing element, such as a blood glucose test element, and other information over a network area so that one or more blood glucose meters remotely receive and are configured to include the calibration data and other information in memory when the meter is subsequently used by the user. User interaction with the blood glucose meter to enter calibration data is eliminated, minimizing the potential for user entry errors and lack of entry of the calibration data by the user, thus decreasing the likelihood for analyte measurement errors. Also eliminated are steps taken by the manufacturer during the manufacture of the analyte test elements to determine and provide the calibration data with the test elements, reducing manufacturing time and costs.

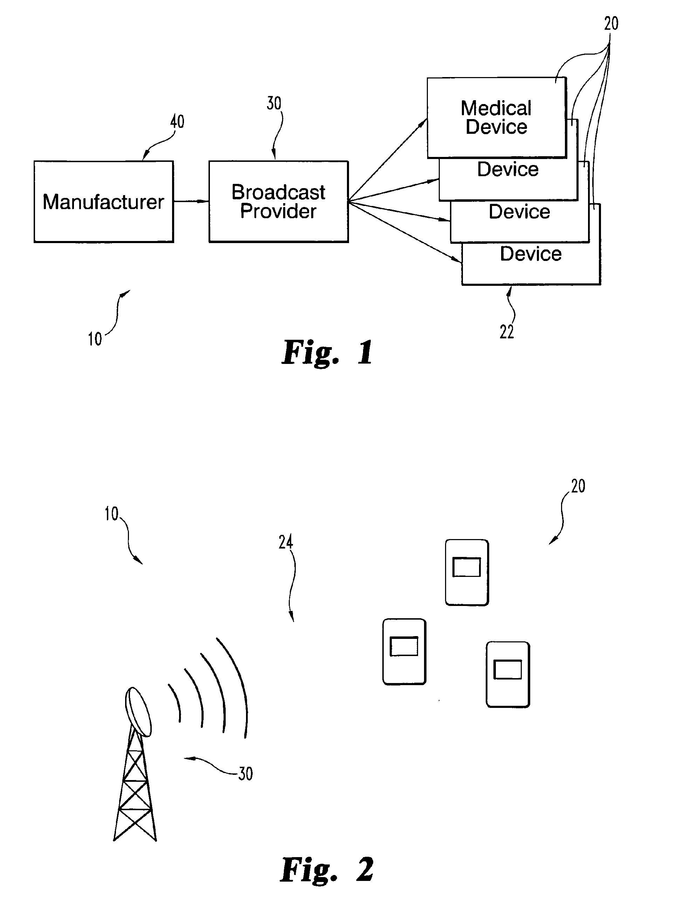

Referring to Figures 1 and 2, the system 10 includes a data receiver and decoder module 22 within a population of medical devices 20, a data transmission network module (DTM) 30 with a broadcast provider to broadcast data signals over a network coverage area 24, and a data provision module 40 to transfer medical device data from the medical device manufacturer (MDM) to the broadcast provider for broadcast to medical devices 20. As used herein, the MDM may be the manufacturer of the medical device, the manufacturer of ancillary or auxiliary devices that are used by the medical device, or third parties associated with the manufacturers of the medical devices and/or ancillary components of the medical devices.

In data provision module 40, the MDM creates medical device data that is required or useful to all or a portion of medical devices 20, such as programming data, configuration or memory updates, product recall data, warnings or operating instruction data, educational data, motivational data, or commercial data, for example. In one specific example for medical devices that are blood glucose meters, calibration data for analyte test elements is provided for a lot of just-manufactured test elements. Examples of calibration data include a strip lot number, a meter ID number, and calibration parameters for that specific test element lot. It is also contemplated that analyte test element data may include one or more of a determination that a lot of test elements is no longer usable or is recalled, or that a lot of test elements has an extended useful life. Additional examples of medical device data that can be required or useful to all or a portion of medical devices 20 are provided in U.S. Patent No. 5,366,609 to White et al., which is incorporated herein by reference in its entirety. Such examples include stored parameter values; procedure routines to control operation of the medical device; testing procedure routines; constants and other data directed to specific batches of test elements for carrying out analyte determination procedures, such as measurement delay times, incubation times, the number of measurements to be taken during a measurement period, testing threshold levels form comparison to actual test results, conversion factors, and failsafe test threshold values.

The information useful to one or more of medical devices 20 is transmitted by the MDM to a broadcast provider of DTM 30 by conventional means, such as a dedicated computer link, internet transfer, telephone lines, satellite, a wireless link, or other suitable communications link. The broadcast provider of DTM 30 prepares the medical device data for broadcast by encoding, compression, packetization, synchronization, randomization, interleaving, or other method generally in use or unique to the particular broadcast format. The broadcast provider of DTM 30 then broadcasts data signals over its network area to transmit the medical device data to all medical devices 20 within range of the network area.

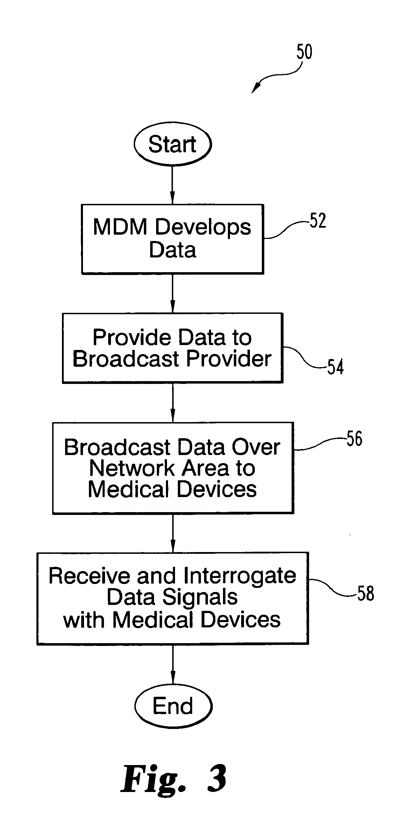

Referring to Figure 3, a flow chart 50 illustrates steps included in a method for broadcasting data signals to a plurality of medical devices 20 distributed over a network area. At step 52, the MDM develops medical device data associated with all of or specific classes of the medical devices 20, or medical device data associated with peripheral or ancillary devices used by all or certain classes of the medical devices 20. For example, the MDM may develop new operating parameters or updates in existing operating parameters for some or all of the medical devices 20. The operating parameters may include new software to control the operation of a processor to perform specific functions, updates to existing software, or updates in operating instructions for the processor of the medical device. The MDM may also develop medical device data that is useful to the users of all or a portion of the medical devices 20 in the network area. Such medical device data may include treatment information, recall and expiration information, warranty information, motivational information, commercial information such as rebates or product and software upgrades and updates, information pertaining to treatment providers, and other suitable information relating to medical devices 20 or the users thereof.

Medical device data may further include other larger data streams that carry signals for instructional or other videos as well as user manual downloads. Because of the significantly larger amount of data, these types of medical device data need not be saved or stored by the medical device, but rather may be merely decoded and displayed using temporary or otherwise short-term, dynamic memory stores.

At step 54 the MDM provides the medical device data to the broadcast provider. The medical device data can be provided by any suitable means, including electronically, textually or graphically. The broadcast provider then, if necessary, formats and/or encodes the medical device data for broadcast over the network area. Alternatively, the MDM may provide the medical device data in a formatted or encoded condition to the broadcast provider at step 54.

At step 56, the broadcast provider broadcasts data signals to transmit the medical device data over the network area to medical devices 20. As discussed further below, the network area can be local, national, or worldwide, depending on the broadcast system that is employed. The medical devices 20 within the network area each include a receiver that is configured to receive the data signals from the broadcast provider and a decoder, if necessary, to decode any encoded medical device data included with the data signals.

At step 58, each of the medical devices 20 operates in a receiving mode at some point in time during the broadcast of the data signals to receive and interrogate the data signals and determine whether the data signals are relevant to that particular medical device 20. Not all broadcast signals need to be directed to every medical device 20 within the network area. For example, the broadcast signal may only be relevant to a certain class or classes of medical devices 20, and the receivers of medical devices 20 are programmed to interrogate the data signals to determine whether or not to ignore the data signal. If the receiver determines the broadcast signal is relevant to that medical device 20, then the medical device 20 is operable to receive and apply the medical device data transmitted with the data signal, as discussed further below.

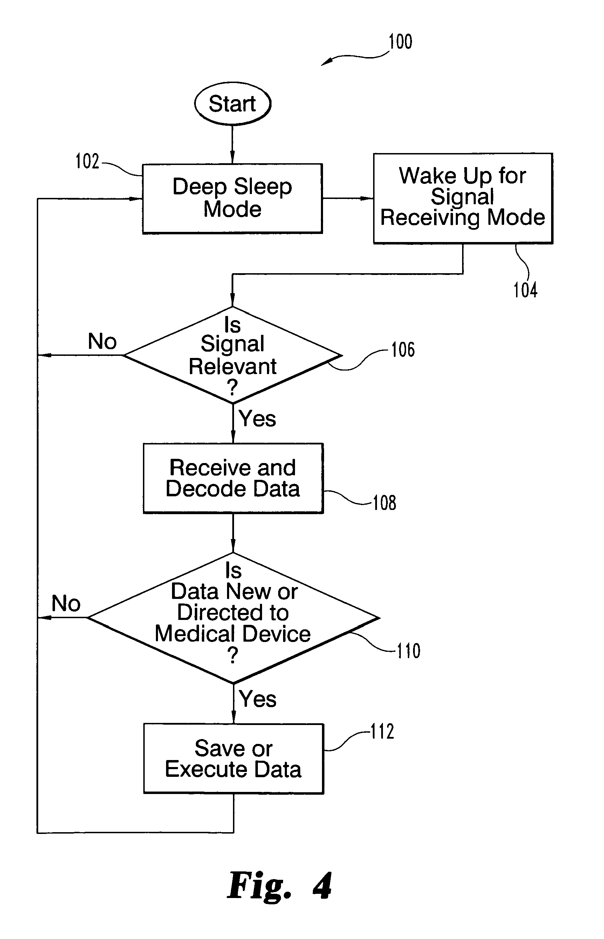

Referring now to Figure 4, a flow chart 100 is provided to illustrate a method of operation for medical devices 20 within the network area. Medical devices 20 are configured to normally operate in a deep sleep mode 102 when not in use to preserve battery power. Medical devices 20 are programmed to periodically wake up from their deep sleep mode 102 for operation in a light sleep or receiving mode at step 104 in order to receive data signals from the broadcast provider. Operation in the receiving mode can be performed automatically at specific or random time intervals by medical devices 20 without any user prompting, although user prompting of medical devices 20 to operate in the receiving mode is not prohibited. If the medical device 20 is in use during its time to receive signals, the medical device 20 can be programmed to defer operation in the receiving mode until its use is complete, or to interrupt its current operation to operate in the receiving mode to receive and interrogate data signals.

In receiving mode 104, each of the medical devices 20 is operable to interrogate the data signal at step 106 to determine if the data signal that is received from the broadcast provider over the network area is directed to that medical device 20. If the interrogation reveals the data signal detected is not directed to a particular one or more of medical devices 20, those medical devices 20 ignore the data signal and return to operation in a deep sleep mode at step 102 for a period of time until waking up again for operation in the receiving mode at step 104.

If the interrogation at step 106 indicates a data signal that is relevant to one or more of the medical devices 20, those one or more medical devices 20 operate to receive and decode the relevant medical device data encoded with the data signal at step 108. The medical device data transmitted with the data signal is analyzed by the processor of the medical devices 20 at step 110 to determine if the medical device data is new data, medical device data associated with that medical device 20 or its device class, or if the medical device data is associated with the product type, MDM or provider group associated with that medical device 20. If at step 110 it is determined that the medical device data is not new, or not otherwise directed to or intended for that medical device 20, that medical device 20 returns to operation in deep sleep mode at step 102 for a period of time until it wakes up again for operation in the receiving mode at step 104. If the medical device data is new or otherwise directed to or intended for that medical device 20, medical device 20 saves the medical device data to memory or executes the medical device data to update operating parameters at step 112. The medical device 20 then returns to operation in a deep sleep mode at step 102 if it is not in current use by the user.

It is further contemplated that medical device 20 can be programmed and operable to verify the medical device data received from the broadcast network to ensure the medical device data is accurate and/or not corrupt. If the medical device data is not verified to be accurate and/or non-corrupt, then the medical device data is ignored, or at least not saved to memory or executed upon by medical device 20. The data verification can be performed in conjunction with the interrogation of the medical device data or as an additional step before or after interrogation of the medical device data.

Various techniques are contemplated to verify the medical device data received by medical device 20. One data verification procedure involves a checksum, in which a data packet is processed by convolution with a key. The sum of all the convoluted values in the data packet is determined, and the determined value and convoluted values are sent along with the medical device data. The receiving medical device calculates the sum of the convoluted values according to the same algorithm. If the calculated value does not match the determined value provided with the key, the associated medical device data is corrupted or inaccurate and disregarded by medical device 20.

In another procedure, redundant transmission of the medical device data is provided. The same medical device data is broadcast and transmitted multiple times, and all the medical device data received by the medical device must match a previously received set or segment of medical device data before the medical device data is accepted and acted upon by medical device 20. In situations where the broadcast network continuously broadcasts medical device data over the network area, it is contemplated that particular sets or segments of the medical device data are broadcast continuously for a period of time or a predetermined number of times over the network area and are thus receivable multiple times by the medical devices 20 in the network area. The medical devices 20 can be programmed to not perform any operations or store any of the medical device data in memory until the same set or segment of medical device data is received multiple times by the medical device. Medical device 20 may further be programmed to ignore transmissions of medical device data once that same medical device data has been successfully implemented and/or stored by medical device 20.

In another approach for verifying the medical device data, the medical device data is broadcast and transmitted in multiple formats, such as in binary and in ASCII formats. Medical device 20 accepts the data only when the values in each format match one another. It is further contemplated that any combination of data verification methods can be employed to increase confidence in the data accuracy. Furthermore, data verification techniques for the medical device data are not limited by the above examples, and may include any suitable data verification technique known in the art.

To conserve power during its normal operation, medical device 20 may wake up at some user interaction in order to receive broadcast signals rather than wake up at pre-defined time intervals as discussed above. In one specific example, a medical device 20 that is a blood glucose meter wakes up from its deep sleep mode at step 102 when the user inserts an analyte test element into a testing port or attempts to take a reading from a test element with the blood glucose meter. The processor of the blood glucose meter queries the test element for a type or lot identifier associated with the test element, and then searches the internal memory of the blood glucose meter to locate, for example, a set of calibration values or operational sequence instructions related to or specifically designated for the lot or identifier of the test element. If the type or lot identifier of the test element is resident in the memory of the blood glucose meter, the blood glucose test proceeds immediately. If not resident in memory, the blood glucose meter activates its receiver and searches for information relevant to the test element so the calibration data, operational sequence instructions, or other medical device data associated with the test element can be received from the broadcast provider and used by the blood glucose meter to perform the test.

In another embodiment, medical device 20 includes a detection device to detect information encoded on an ancillary component of medical system 10, such as a bar code reader to read a bar code on an analyte test element. The bar code on the ancillary component transmits to medical device 20 a lot identification code or other useful information associated with that component. Medical device 20 may then check its internal memory for medical device data relevant to that component based on its lot identification code or other information that is detected. If the relevant medical device data is located in internal memory, medical device 20 uses the stored information to provide its processor operating parameters, calibration data or other information that facilitates use of the ancillary component by medical device 20. If the relevant medical device data is not stored or resident in the internal memory of medical device 20, then medical device 20 activates its receiver to receive data signals broadcast over the network area and to decode any data signals that are relevant to the operation of the ancillary component with the medical device 20.

In one embodiment, the receiving, analyzing, and saving or execution of instructions in the medical device data transmitted by the data signals is performed seamlessly by medical device 20 so that no user interaction is required with medical device 20 to complete these steps. Medical device data is broadcast primarily in a one-directional mode from the broadcast provider over the network area to the medical devices 20 in a transparent and seamless manner to the user and without requiring the user to interact with the medical device, the broadcast network system, or the MDM to receive, analyze and apply the data signals. However, user interaction with medical device 20 in completing one or more of these steps is not prohibited. After medical device 20 receives the data signals and writes the medical device data transmitted therewith to memory or executes instructions included in the medical device data, when the user next turns on medical device 20 it will include the updated operating parameters or configuration, or the new medical device data or instructions resident in its memory for use by medical device 20 or for display thereby.

In one specific embodiment associated with a medical device 20 that is a blood glucose meter, before the MDM provides a lot of analyte test elements such as blood glucose test strips for shipment, the test elements are encoded with a lot-specific identifier during manufacture. Thus, the test element carries a lot identifier or other key to identify the manufacturing lot of test elements to which it belongs, and this identification number can be assigned and placed on or with the test element early in the manufacturing process. In contrast, current techniques for providing calibration data for analyte test elements are directed to increasing the amount of calibration data that can be determined by the blood glucose meter directly from the test element. However, the calibration data placed on the test elements must be determined and associated with the test element prior to the MDM releasing the test element into distribution channels, increasing manufacturing time and cost.

Lot specific information for each lot of test elements, such as the calibration data, is determined by the MDM. The MDM provides the lot specific information to the broadcast provider for broadcast over the network area. This calibration data is then broadcast over the network area and received by the receiver of the blood glucose meter and the other meters in the network typically before the test elements become available to the users. The receivers of each of the blood glucose meters detect the data signals and, if relevant to that blood glucose meter, store the calibration data transmitted therewith in the memory of that blood glucose meter. Each blood glucose meter is programmed to determine the lot identification of a test element when that test element is inserted into or read by the meter. The calibration data for the lot-specific identifier with which the test element is associated is then identified from memory of the meter, and used to calibrate the meter accordingly to take a proper reading from the test element that is being read by the meter. If the calibration data for the test element that is being read is not available from the memory of the blood glucose meter, the meter is programmed to receive data signals from the broadcast network system and identify and receive signals with calibration data associated with the lot-specific identifier of the inserted test element.

Since each blood glucose meter in the network either has calibration data stored in memory for test element lots that are usable by that meter or obtains the calibration data from the broadcast network system, any test element that is purchased from the MDM can be used with updated calibration data. The blood glucose meter can be programmed so that if the calibration data is not stored in its memory or available from the network, then the test element is not properly usable by the blood glucose meter, and the blood glucose meter can be programmed so that no reading is taken from an improper test element. For example, when a particular test element lot expires or is recalled, the broadcast network system can broadcast a signal with an instruction that is received by the blood glucose meter to erase calibration data for that particular lot of test elements from its memory so that the user cannot use an improper test element. Furthermore, since the calibration data for all available test element lots will be resident on the memory of the meter or available from the network, there is no need for the MDM to provide calibration data on the test elements or to provide a key with a package of test elements that includes the calibration data.

The present invention allows the MDM to provide useful information to the user of the medical device very soon after the information is known to the MDM and without placing or storing this information in the medical device or its ancillary components prior to shipment. Even after distribution to the end user, the medical device remains updated with the latest information regarding its operation and components incorporated into its processing systems or stored in its memory with no user interaction with the medical device or broadcast network system required. In addition, the receipt and updating of information stored in the medical device or update of operational instructions received from the medical device data transmitted by the data signals over the network area occurs seamlessly and transparently to the user without any user interaction with the medical device required.

Other uses for the broadcast network system and medical devices 20 with the receiver/decoder are also contemplated. For example, the broadcast network system can be used to broadcast data signals that automatically set the date and time of the medical device 20 according to local conditions. The network may also transmit medical data relating to treatment plans, motivating and educating the user to perform tests using medical device 20 and to take appropriate steps to address any problems identified in the tests, to communicate recalls of medical devices 20 or recalls relating to ancillary components used with medical devices 20, and to provide warnings and notices relating to one or more medical devices 20 specifically or to treatment of medical conditions associated specifically with the user of one or more of medical devices 20. Non-medical information such as commercial offers and discounts may also be broadcast to medical devices 20. The broadcast network system may also wirelessly transmit device-specific data to one or more medical devices 20, including targeted medical device alerts, configuration data, user interface options, or updates to all or a portion of an operating system of the targeted medical device or devices 20.

Additional examples of the information that can be provided to one or more of medical devices 20 include, but are not limited to: new images and logos; updated contact information for local health care providers or the MDM; warnings or advice, such as test element recalls or test element expiration; advertisements or coupons relating to, for example, special product pricing, new product announcements, and supplemental information relating to diet, exercise or auxiliary products; and notices for local conferences, support groups, or websites for disease related information; and data analysis and support. The broadcast network system may also signal a medical device 20 or group of medical devices 20 that a firmware update or reconfiguration is available through another channel or means, prompting the user to connect to a website or other by-directional wired or wireless medium suited for downloading, installing, configuring and confirming the medical device's application or operating mode.

Various configurations for the broadcast network system are contemplated. The broadcast network system may include satellite transmitters that cover large areas of territory or one or more local transmission sources, such as may be found with a pager network. For example, the network service MYAMBIENT has a pager network for wirelessly transmitting time and date, weather and other local information to receiver-enabled devices. This type of broadcast network system can be configured to transmit additional medical device data specifically adapted for receipt by the medical devices 20 within its network area. Other options for wirelessly transmitting medical device data for medical devices 20 include Bluetooth, Zigbee, Wibree, ultra-wide band (UWB), wireless local area network (WLAN), General Packet Radio Service (GPRS), Worldwide Interoperability for Microwave Access (WiMAX or WiMAN), Wireless Medical Telemetry (WMTS), Wireless Universal Serial Bus (WUSB), Global System for Mobile communications (GSM), WLAN 802.11 x standards, and any of the series of third-generation (3G) mobile phone standard technology. The broadcast network system may also employ any one or more of higher bandwidth broadcast radio (FM, HD, Digital Audio Broadcasting (DAB), Digital Radio Mondiale (DRM)), broadcast DTV (DVB-H, ISDB-T, Digital Multimedia Broadcasting (DMB)), or pager networks such as FLEX, ReFLEX, POCSAG (Post Office Code Standardization Advisory Group). Ultra low frequency bands may also be used for wider coverage with fewer repeaters.

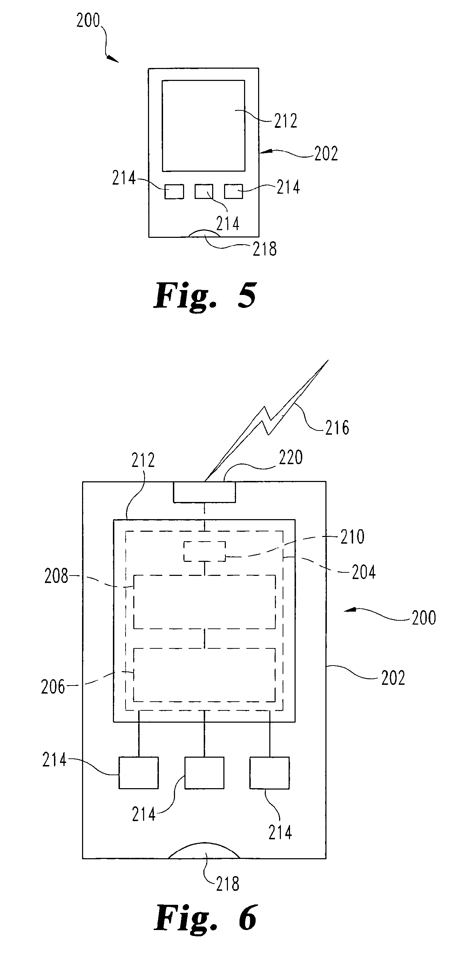

Referring to Figure 5 there is shown a specific example of a medical device 20 in the form of a blood glucose (bG) meter 200 for testing and measuring blood glucose levels in the blood of an individual. It should be understood, however, the present invention has application with medical devices other than blood glucose meters. In the specific embodiment of Figure 5, the bG meter 200 includes a housing 202 with a capable display 212, a user entry means 214, and a test element port 218. Electronic circuitry is contained within housing 202 to provide an electrochemical or photochemical measurement of a glucose level from a sample of blood on a test element inserted into test element port 218. Housing 202 can be sufficiently compact so that it can be conveniently hand held and carried by the user. The bG meter 200 may also include one or more other compartments or features for storage of lancets, navigation pens, or other devices (not shown) which may be useful with bG meter 200.

In Figure 6 there is shown one embodiment of a schematic of bG meter 200. The bG meter 200 includes a controller 204, memory 206 associated with controller 204, a programmable processor 208 associated with controller 204 and connected with memory 206, and a real-time clock 210 associated with controller 204 and connected with processor 208. The bG meter 200 also includes a receiver/decoder 220 operably coupled with controller 204 so that data signals received from the broadcast network system can be interrogated and subsequently written to memory 206, used by processor 208 to perform an operation or update operating parameters, or ignored. Display 212 is connected with processor 208 with, for example, a display driver, and operable to provide a user readable display of output from processor 208. User entry means 214 is connected with processor 208 and accessible by the user to provide input to processor 208. Processor 208 is connected with test element port 218 and operable to process and record medical device data in memory 206 relating to a blood glucose measurement taken in test element port 218 and produce a representation of the current bG measurement and associated data on display 212. Processor 208 is further programmable to receive input commands from user entry means 214 and provide an output that responds to the input commands.

Controller 204 may be comprised of one or more components configured as a single unit or of multi-component form. Controller 204 may be programmable, a state logic machine or other type of dedicated hardware, or a hybrid combination of programmable and dedicated hardware. One or more components of controller 204 may be of the electronic variety defining digital circuitry, analog circuitry, or both. As an addition or alternative to electronic circuitry, controller 204 may include one or more mechanical or optical control elements.

In one embodiment including electronic circuitry, controller 204 includes an integrated processor 208 operatively coupled to one or more solid-state memory devices defining, at least in part, memory 206. For this embodiment, memory 206 contains operating logic to be executed by a processor 208 that is a microprocessor and is arranged for reading and writing of medical device data in memory 206 in accordance with one or more routines of a program executed by microprocessor 208.

Memory 206 may include one or more types of solid-state electronic memory and additionally or alternatively may include the magnetic or optical variety. For example, memory 206 may include solid-state electronic Random Access Memory (RAM), Sequentially Accessible Memory (SAM) (such as the First-In, First-Out (FIFO) variety or the Last-In First-Out (LIFO) variety), Programmable Read Only Memory (PROM), Electrically Programmable Read Only Memory (EPROM), or Electrically Erasable Programmable Read Only Memory (EEPROM); or a combination of any of these types. Also, memory 206 may be volatile, nonvolatile or a hybrid combination of volatile and nonvolatile varieties. Some or all of memory 206 can be of a portable type, such as a disk, tape, memory stick, cartridge, or the like. Memory 206 can be at least partially integrated with processor 208 and/or may be in the form of one or more components or units.

Besides memory 206, controller 204 may also include clock 210, display 212, and entry means 214 associated therewith, along with signal conditioners, filters, limiters, Analog-to-Digital (A/D) converters, Digital-to-Analog (D/A) converters, communication ports, or other types of operators as would occur to those skilled in the art to implement the present invention. Entry means 214 may include one or more input devices like a pushbutton, keyboard, mouse or other pointing device, touch screen, touch pad, roller ball, or a voice recognition input subsystem. Display 212 may include one or more output means like an operator display that can be of a Cathode Ray Tube (CRT) type, Liquid Crystal Display (LCD) type, plasma type, Organic Light Emitting Diode (OLED) type, a printer, or the like. Other input and display means can be included such as loudspeakers, voice generators, voice and speech recognition systems, haptic displays, electronic wired or wireless communication subsystems, and the like.

A communications link 216 for wired or wireless connection of receiver/decoder 220 with the broadcast network system and/or a secondary device, such as a personal computer, modem, local area network, or the worldwide web is also provided and operably coupled to receiver/decoder 220. Data signals from the broadcast network system are received by receiver/decoder 220 and interrogated for applicability to bG meter 200. If the data signal is determined to be applicable to bG meter 200, the data signal is transmitted to controller 204 for subsequent analysis. For example, the data signal may include medical device data to update the operating system, the time, or other internal programming of processor 208. In another example, the signal may include medical device data for subsequent display to the user on display means 212, such as treatment information, promotional information, motivational information, or updates regarding the use and operation of bG meter 200. In another example, the data signal may include medical device data for storage in memory 206 that is subsequently accessed by processor 208 during operation, such as calibration data, expiration data, or recall data associated with one or more lots of test elements. In still another example, the medical device data may be redundant or not applicable to the particular blood glucose meter 200 and ignored after this determination is made.

While the invention has been illustrated and described in detail in the drawings and foregoing description, the same is to be considered as illustrative and not restrictive in character, it being understood that only certain embodiments have been shown and described and that all changes and modifications that come within the spirit of the inventions are desired to be protected. It should be understood that while the use of words such as preferable, preferably, preferred or more preferred utilized in the description above indicate that the feature so described may be more desirable, it nonetheless may not be necessary and embodiments lacking the same may be contemplated as within the scope of the invention, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

The following is a list of preferred embodiments of the invention:
1. A system for configuring medical devices, comprising:
   a plurality of portable medical devices distributed over a network area, each of said medical devices including a receiver to receive data signals when said data signals are broadcast over said network area, each of said medical devices further including a memory and a processor operably linked with said receiver; and
   a broadcast network system for broadcasting medical device data transmitted by said data signals in said network area for receipt by said receivers of each of said plurality of medical devices in said network area, wherein said plurality of medical devices each include means operable upon receiving said data signals for at least one of updating operating parameters of said processor in accordance with said medical device data and storing said medical device data in said memory.
2. The system of embodiment 1, wherein said broadcast network system includes a broadcast provider linked to said broadcast network system, and further comprising a medical device manufacturer for supplying said medical device data to said broadcast provider, wherein said broadcast provider formats said medical device data for wireless transmission in said network area.
3. The system of embodiment 2, further comprising:
   a communications link between said medical device manufacturer and said broadcast provider; and
   said medical device data is formatted by said broadcast provider by at least one of encoding, compression, and packetization of said medical device data for broadcast with said data signals over said network area.
4. The system of embodiment 1, wherein said medical device data include calibration data for at least one lot of analyte test elements.
5. The system of embodiment 4, wherein said calibration data include a test element lot number and calibration parameters associated with said test element lot number.
6. The system of embodiment 5, wherein at least a portion of said plurality of medical devices receive said data signals and store said calibration data associated with said data signals in said memory thereof, wherein said processors of said portion of said medical devices are operable to automatically associate said stored test element lot number to a lot number of an analyte test element detected by said medical devices and calibrate said medical devices with said calibration parameters of said associated test element lot number.
7. The system of embodiment 6, wherein said memory of said portion of said medical devices includes calibration data received from said broadcast network system for a plurality of lots of analyte test elements.
8. The system of embodiment 1, wherein said medical device data include at least one of treatment information, medical device information, warning information, promotional information, educational information and motivational information.
9. The system of embodiment 8, wherein said processor is operable to display said medical device data on said medical device when said medical device is accessed by the user and said medical device data is stored in said memory of said medical device.
10. The system of embodiment 1, wherein said broadcast network system includes at least one satellite transmitter.
11. The system of embodiment 1, wherein said broadcast network system includes at least one local transmitter that is fixed in location.
12. The system of embodiment 1, wherein said receiver is operable to automatically receive said data signals when said data signals are broadcast over said network area and said medical device is in said network area.
13. The system of embodiment 12, wherein said processor is operable to automatically perform an operation with said medical device data transmitted with said data signals without user interaction with said medical device.
14. The system of embodiment 1, wherein said data signals are radio frequency signals.
15. A portable blood glucose meter, comprising:
   a housing including a display and a user entry means for receiving user input;
   a memory and a processor in said housing operably connected to said display and said user entry means;
   a port including circuitry for determining a value representative of a property of a fluid sample carried by an analyte test element inserted in said port and providing said value to said processor; and
   a receiver operable to seamlessly receive data signals from a broadcast network system, wherein said receiver is linked to said processor and said memory and said processor is operable to seamlessly process medical device data transmitted with said data signals from said receiver in order to at least one of update operating parameters of said processor with said medical device data and store said medical device data in said memory.
16. The meter of embodiment 15, wherein said receiver includes a decoder and said receiver and decoder are operable to receive and decode said data signals.
17. The meter of embodiment 15, wherein said processor is operable to update operating instructions of said processor with said medical device data transmitted with said data signals.
18. The meter of embodiment 15, wherein said processor is operable to update software stored in said memory with said medical device data transmitted with said data signals.
19. The meter of embodiment 15, wherein said medical device data transmitted with said data signals includes calibration data for at least one lot of analyte test elements and said processor is operable to store said calibration data in said memory.
20. The meter of embodiment 19, wherein said processor is operable to associate said calibration data stored in said memory to an analyte test element inserted in said port.
21. A method for updating a medical device, comprising:
   providing a broadcast network system with a network area;
   broadcasting data signals over the network area to a plurality of medical devices in the network area, wherein each of the plurality of medical devices receives the data signals;
   interrogating the data signals with each of the medical devices; and
   performing an operation with each of the medical devices based on the interrogation of the data signals.
22. The method of embodiment 21, wherein performing the operation with each of the medical devices includes at least a portion of the medical devices disregarding the data signals.
23. The method of embodiment 21, wherein performing the operation with each of the medical devices includes at least a portion of the medical devices updating an operating system thereof with medical device data transmitted with the data signals.
24. The method of embodiment 21, wherein performing the operation with each of the medical devices includes at least a portion of the medical devices storing medical device data transmitted with the data signals in a memory of the portion of the medical devices.
25. The method of embodiment 21, wherein performing the operation with each of the medical devices includes at least one of: disregarding the data signals, updating an operating system of the medical devices with medical device data transmitted with data signals, and storing medical device data transmitted with the data signals in a memory of the medical devices.
26. The method of embodiment 21, wherein each of the plurality of medical devices seamlessly receives the data signals when the medical device is located in the network area.
27. The method of embodiment 21, wherein the data signals are formatted for wireless transmission over the network area.
28. The method of embodiment 21, further comprising:
   providing medical device data to a broadcast provider, wherein the medical device data is provided by a manufacturer of the plurality of medical devices; and
   formatting the medical device data for transmission over the network area with the data signals.
29. The method of embodiment 21, wherein the data signals include calibration data for a manufactured lot of analyte test elements.
30. The method of embodiment 29, wherein the calibration data includes one or more of an analyte test element lot number and calibration parameters associated with the analyte test element lot number.
31. The method of embodiment 30, further comprising:
   storing the calibration data in a memory of at least a portion of the plurality of medical devices;
   associating the stored analyte test element lot number to a lot number of an analyte test element detected by a corresponding one of the portion of medical devices;
   calibrating the corresponding medical device with the calibration parameters of the associated stored analyte test element lot number; and
   testing an analyte with the detected analyte test element using the calibrated medical device.
32. The method of embodiment 31, wherein the memory of the calibrated medical device includes calibration data for a plurality of lots of analyte test elements and the associated stored analyte test element lot number is selected from the calibration data of the plurality of lots of analyte test elements stored in the memory.
33. The method of embodiment 21, wherein the data signals include at least one of expiration data for a lot of analyte test elements and recall data for a lot of analyte test elements.
34. The method of embodiment 21, wherein:
   the medical device data is transmitted by the data signals includes at least one of treatment information, medical device information, warning information, promotional information, and motivational information;
   performing the operation with each of the medical devices includes at least a portion of the medical devices storing the medical device data transmitted with the data signals in a memory of the portion of the medical devices, and further comprising:
      displaying the medical device data on a display of a corresponding one of the medical devices when subsequently accessed by a user of the medical device.
35. The method of embodiment 21, wherein the data signals include at least one medical device identification number to which the data signals are directed.
36. The method of embodiment 21, wherein performing the operation includes automatically setting a time and a date on a clock of the medical device.
37. The method of embodiment 21, further comprising verifying the medical device data before performing the operation.
38. The method of embodiment 21, wherein verifying the medical device data includes limiting the medical device data to medical device data that matches a previously received segment of medical device data.

## Claims

1. A system for configuring medical devices, comprising:
a plurality of portable medical devices distributed over a network area, each of said medical devices including a receiver to receive data signals when said data signals are broadcast over said network area, each of said medical devices further including a memory and a processor operably linked with said receiver; and
a broadcast network system for broadcasting medical device data transmitted by said data signals in said network area for receipt by said receivers of each of said plurality of medical devices in said network area, wherein said plurality of medical devices each include means operable upon receiving said data signals for at least one of updating operating parameters of said processor in accordance with said medical device data and storing said medical device data in said memory.

2. The system of claim 1, wherein said broadcast network system includes a broadcast provider linked to said broadcast network system, and further comprising a medical device manufacturer for supplying said medical device data to said broadcast provider, wherein said broadcast provider formats said medical device data for wireless transmission in said network area.

3. The system of claim 2, further comprising:
a communications link between said medical device manufacturer and said broadcast provider; and
said medical device data is formatted by said broadcast provider by at least one of encoding, compression, and packetization of said medical device data for broadcast with said data signals over said network area.

4. The system of claim 1, wherein
said medical device data include calibration data for at least one lot of analyte test elements; said calibration data include a test element lot number and calibration parameters associated with said test element lot number.

5. The system of claim 4, wherein at least a portion of said plurality of medical devices receive said data signals and store said calibration data associated with said data signals in said memory thereof, wherein said processors of said portion of said medical devices are operable to automatically associate said stored test element lot number to a lot number of an analyte test element detected by said medical devices and calibrate said medical devices with said calibration parameters of said associated test element lot number.

6. The system of claim 1, wherein said medical device data include at least one of treatment information, medical device information, warning information, promotional information, educational information and motivational information.

7. The system of claim 1, wherein said receiver is operable to automatically receive said data signals when said data signals are broadcast over said network area and said medical device is in said network area.

8. The system of claim 7, wherein said processor is operable to automatically perform an operation with said medical device data transmitted with said data signals without user interaction with said medical device.

9. A portable blood glucose meter, comprising:
a housing including a display and a user entry means for receiving user input;
a memory and a processor in said housing operably connected to said display and said user entry means;
a port including circuitry for determining a value representative of a property of a fluid sample carried by an analyte test element inserted in said port and providing said value to said processor; and
a receiver operable to seamlessly receive data signals from a broadcast network system, wherein said receiver is linked to said processor and said memory and said processor is operable to seamlessly process medical device data transmitted with said data signals from said receiver in order to at least one of update operating parameters of said processor with said medical device data and store said medical device data in said memory.

10. The meter of claim 9, wherein said receiver includes a decoder and said receiver and decoder are operable to receive and decode said data signals.

11. The meter of claim 9, wherein said processor is operable to update operating instructions of said processor or software stored in said memory with said medical device data transmitted with said data signals.

12. The meter of claim 9, wherein said medical device data transmitted with said data signals includes calibration data for at least one lot of analyte test elements and said processor is operable to store said calibration data in said memory, and
wherein said processor is operable to associate said calibration data stored in said memory to an analyte test element inserted in said port.

13. A method for updating a medical device, comprising:
providing a broadcast network system with a network area;
broadcasting data signals over the network area to a plurality of medical devices in the network area, wherein each of the plurality of medical devices receives the data signals;
interrogating the data signals with each of the medical devices; and
performing an operation with each of the medical devices based on the interrogation of the data signals,.
wherein performing the operation with each of the medical devices includes at least a portion of the medical devices disregarding the data signals or includes at least a portion of the medical devices updating an operating system thereof with medical device data transmitted with the data signals or
includes at least a portion of the medical devices storing medical device data transmitted with the data signals in a memory of the portion of the medical devices.

14. The method of claim 13, further comprising:
providing medical device data to a broadcast provider, wherein the medical device data is provided by a manufacturer of the plurality of medical devices; and
formatting the medical device data for transmission over the network area with the data signals.

15. The method of claim 13, wherein the data signals include calibration data for a manufactured lot of analyte test elements, and/or at least one medical device identification number to which the data signals are directed, and/or at least one of expiration data for a lot of analyte test elements and recall data for a lot of analyte test elements; and.
wherein the calibration data includes one or more of an analyte test element lot number and calibration parameters associated with the analyte test element lot number.

16. The method of claim 15, further comprising:
storing the calibration data in a memory of at least a portion of the plurality of medical devices;
associating the stored analyte test element lot number to a lot number of an analyte test element detected by a corresponding one of the portion of medical devices;
calibrating the corresponding medical device with the calibration parameters of the associated stored analyte test element lot number; and
testing an analyte with the detected analyte test element using the calibrated medical device.

17. The method of claim 13, wherein:
the medical device data is transmitted by the data signals includes at least one of treatment information, medical device information, warning information, promotional information, and motivational information;
performing the operation with each of the medical devices includes at least a portion of the medical devices storing the medical device data transmitted with the data signals in a memory of the portion of the medical devices, and further comprising:
displaying the medical device data on a display of a corresponding one of the medical devices when subsequently accessed by a user of the medical device.

18. The method of claim 13, wherein performing the operation includes automatically setting a time and a date on a clock of the medical device.
